Europäisches Patentamt

European Patent Office

Office européen des brevets

(11) Numéro de publication: **0 136 337**
**B1**

(12) **FASCICULE DE BREVET EUROPEEN**

(45) Date de publication du fascicule du brevet:
07.10.87

(21) Numéro de dépôt: 84901082.2

(22) Date de dépôt: 09.03.84

(86) Numéro de dépôt international:
PCT/FR 84/00054

(87) Numéro de publication internationale:
WO 84/03435 (13.09.84 Gazette 84/22)

(51) Int. Cl.⁴: **A 61 F 9/00**

(54) **DISPOSITIF POUR LA POSE DE LENTILLES DE CONTACT SUR L'OEIL.**

(30) Priorité: 11.03.83 FR 8304065

(43) Date de publication de la demande:
10.04.85 Bulletin 85/15

(45) Mention de la délivrance du brevet:
07.10.87 Bulletin 87/41

(84) Etats contractants désignés:
AT BE CH DE FR GB LI LU NL

(56) Documents cités:
BE - A - 663 413
DE - B - 1 255 345
FR - A - 1 401 116
FR - A - 2 375 004
FR - E - 86 689
US - A - 3 031 918
US - A - 3 934 914
US - A - 4 123 098
US - A - 4 200 320

(73) Titulaire: FAURE, Hubert, 9 avenue Ingres, F-75016 Paris
(FR)

(72) Inventeur: FAURE, Hubert, 9 avenue Ingres,
F-75016 Paris (FR)

(74) Mandataire: Sauvage, Renée, CABINET
SAUVAGE 100 bis, avenue de Saint-Mandé,
F-75012 Paris (FR)

ACTORUM AG

## Description

La présente invention concerne un dispositif pour la pose des lentilles de contact sur l'œil.

On sait qu'une lentille de contact, destinée à la correction d'un défaut de vision, est réalisée en une matière plastique transparente, ayant la forme d'une calotte sphérique destinée à épouser la surface de la cornée, calotte dont les faces ont des rayons de courbure différents et dont l'axe optique doit impérativement être mis en correspondance avec la pupille de l'iris.

Actuellement, les personnes équipées de lentilles de contact doivent poser leurs lentilles en les mettant en équilibre sur la pulpe de l'index d'une main tandis que, de l'autre main, ils maintiennent écartées les paupières de l'œil, puis ils appliquent la lentille sur la cornée en ayant à surmonter les réflexes naturels de défense de l'œil à l'approche d'un corps étranger. L'opération de la dextérité et une accoutumance relativement longue à acquérir et toujours aléatoire.

Ce mode de pose étant particulièrement difficile pour les jeunes enfants, les hyper-nerveux, les personnes âgées et les handicapés moteurs, etc. ..., divers dispositifs d'assistance ont été mis au point dont le plus simple et le plus performant paraît être celui que le Demandeur à lui-même décrit dans FR-A-2525472.

Ce dispositif repose sur le principe de la matérialisation d'un axe optique commun à l'œil à appareiller, à la lentille de contact à poser, et à un point de mire porté par le dispositif lui-même.

A cet effet, un cylindre creux en matière plastique, ou autre, rigide ou semi-rigide préférentiellement opaque, est équipé à une extrémité d'un manchon comportant une cuvette de réception destinée à supporter la lentille à poser, et présente à l'autre extrémité un orifice de visée dont le centre constitue ledit point de mire et qui est pratiqué au centre de la paroi constituant le fond du cylindre.

La pose de la lentille sur le globe oculaire à l'aide de ce dispositif consiste, après avoir mis en place la lentille humidifiée sur la cuvette de réception où elle se maintient appliquée du fait de cette humidification, à présenter l'ensemble face à l'œil à appareiller jusqu'à ce que cet œil puisse voir l'orifice de visée à travers la lentille, axialement audit ensemble. Ainsi, la lentille se trouve très exactement axée par rapport à l'iris.

Il n'y a plus qu'à amener progressivement la lentille vers la cornée, sur laquelle elle s'applique par tension superficielle. Du fait que la cuvette de réception de la lentille consiste en un manchon souple bordé d'une collerette sur laquelle vient reposer ladite lentille, ce manchon souple sert d'amortisseur au moment de la mise en contact de la lentille avec la cornée.

Une action ultérieure des paupières se refermant sur le bord de la collerette assure alors le dégagement automatique du dispositif, cependant que la lentille de contact se maintient appliquée par tension superficielle sur la cornée.

Bien utilisé, ce dispositif est très performant. Néanmoins, il peut se produire des erreurs de parallaxe qui entraînent une présentation de la lentille angulairement, et non pas en parallélisme, avec la cornée. Dans un tel cas, lorsque le bord de la lentille prend contact avec un point du globe oculaire, la faible tension superficielle exercée sur la lentille par le manchon support ne peut empêcher la lentille de basculer de ce support; si, par chance, ce basculement se fait vers le globe oculaire, la tension superficielle générée par celui-ci attire la lentille qui adhère alors à l'œil, mais il y a autant de chance pour que le basculement se fasse vers l'extérieur d'où échec de pose.

La présente invention se propose d'apporter à l'utilisateur la possibilité de contrôler la réalisation du parallélisme d'approche entre la lentille et la cornée, avec une certitude mathématique découlant de l'application de lois de l'optique.

Lors de l'approche de la lentille vers l'œil, le parallélisme entre la lentille et la cornée est réalisé lorsque l'axe de l'appareil muni de la lentille et l'axe visuel de l'œil sont en parfaite coïncidence.

Il serait possible d'améliorer le dispositif objet du FR-A-2525472 précitée en lui incorporant un œilleton de faible diamètre, placé au plus près de la lentille; on augmenterait ainsi la précision d'approche sans toutefois supprimer les erreurs de parallaxe, mais la recherche de l'axe du dispositif à travers deux orifices de petit diamètre serait plus difficile pour des yeux qui exigent une correction.

Selon l'invention, la coïncidence rigoureuse entre l'axe de l'appareil muni de la lentille à poser, et l'axe visuel de l'œil est contrôlée en éliminant toute erreur de parallaxe résultant d'une visée non rigoureuse, en proposant un dispositif comprenant un corps cylindrique terminé, à une extrémité, par un support adapté à recevoir la lentille à poser et, à son extrémité opposée, par un opercule au centre duquel est percé un orifice laissant passer la lumière ambiante, qui se caractérise par le fait que la paroi interne du corps cylindrique est réfléchissante.

A cet effet, la paroi interne peut être revêtue d'un dépôt réfléchissant ou être doublée d'un matériau réfléchissant, ce dépôt ou ce doublage pouvant être réalisé par tous moyens connus convenables dont il existe un large choix.

La paroi réfléchissante peut également être obtenue en utilisant, pour la fabrication du corps cylindrique, un matériau réfléchissant, tel que l'acier inoxydable.

La paroi réfléchissante réfléchit circonférentiellement la lumière provenant de l'orifice de visée percé au centre de la paroi du fond du cylindre et dont le centre matérialise l'un des deux points servant de cible pour établir l'axe du dispositif, l'autre point étant constitué par l'axe de la lentille placé sur son support.

La lumière, ainsi réfléchie, donne de l'orifice de visée par rapport à la paroi du cylindre, une image réelle matérialisée par un anneau lumineux dont le diamètre est fonction des caractéristiques géométriques du cylindre, en particulier de son diamètre intérieur.

Lorsque cette image se trouve amenée à être concentrique à l'orifice de visée lui-même, et uniquement dans ce cas, on a la certitude mathématique que l'axe de l'ensemble du dispositif muni de sa lentille et l'axe visuel de l'œil à équiper sont rigoureusement confondus.

La pose de la lentille peut alors se faire, toutes les conditions de succès étant réunies.

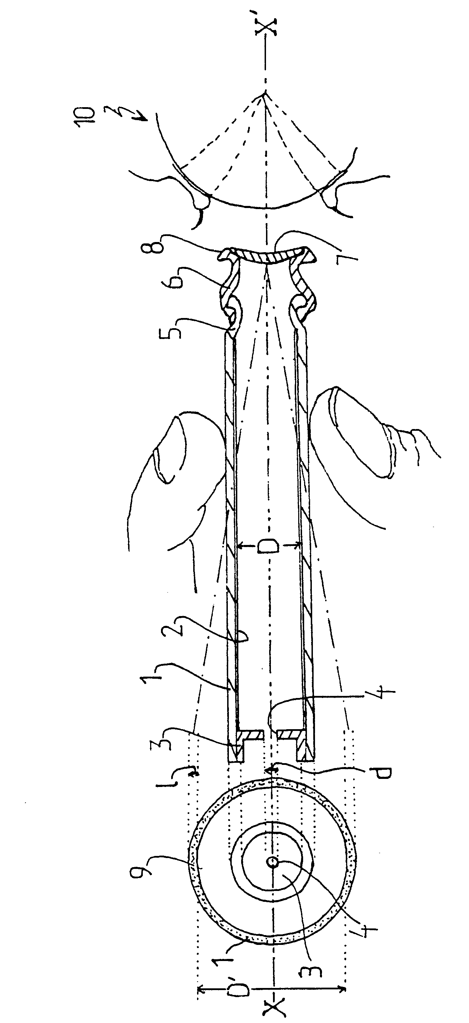

L'invention est décrite en détail ci-après par référence au dessin annexé dans lequel la figure unique montre la position relative correcte du dispositif et de l'œil, et l'image que l'œil reçoit dans ce cas.

Comme il ressort de la figure, le dispositif est constitué d'un corps cylindrique 1, dont la paroi intérieure est pourvue d'un revêtement réfléchissant 2 et qui est fermé à l'une de ses extrémités par un opercule 3 préférentiellement translucide, ce qui a l'avantage d'apprécier le centrage de celui-ci, au milieu de l'anneau lumineux formé 9, comme on le verra plus loin.

Au centre de cet opercule 3 est percé un orifice circulaire 4 de petit diamètre, de l'ordre de 1mm, qui constitue un point de visée pour la matérialisation de l'axe du dispositif et qui laisse pénétrer le flux lumineux provenant de la lumière ambiant environnante.

L'extrémité opposée du corps cylindrique 1 se termine par un embout réducteur 5 adapté à recevoir un manchon amortisseur de réception 6 pour la lentille 7 à poser. La fixation du manchon 6 sur l'embout 5 se fait à frottement doux, le manchon 5 étant constitué d'un matériau souple élastique, de caoutchouc médical par exemple. L'extrémité libre du manchon 6 est terminée par un mince bourrelet de caoutchouc 8 définissant une cuvette formant support pour la lentille 7.

Le diamètre inférieur de l'embout réducteur 5 et le diamètre du manchon 6 doivent être inférieurs au diamètre de la lentille 7, tout en étant d'un diamètre suffisant pour permettre aux rayons visuels passant par la partie optique de la lentille d'explorer le corps cylindrique 1 sans gêner la vision de l'anneau lumineux 9, image de l'orifice 4 donnée par le revêtement réfléchissant 2 de la paroi du corps cylindrique 1.

La lentille 7 est placée sur son support 8 où elle se maintient par l'effet de la tension superficielle, la droite passant par le centre de la lentille 7 et par le centre de l'orifice circulaire 4 ménagé dans l'opercule 3 fermant le cylindre 1, forme alors l'axe x–x' du dispositif.

Le dispositif est utilisé comme suit: l'œil 10 étant maintenu ouvert à l'aide des doigts d'une main, le dispositif, tenu entre les doigts de l'autre main et muni de la lentille 7, est amené à hauteur de l'œil. La vision qu'a cet œil 10 à travers la partie optique de la lentille 7 placée sur son support 8 est celle d'un corps éclairé par le flux lumineux provenant de l'orifice de visée 4 situé sur le fond opposé 3 du dispositif. La lumière issue dudit orifice 4 se réfléchit sur la paroi intérieure réfléchissante 2 du corps cylindrique 1 et donne de cet orifice 4, circonférentiellement, une image réelle 9 qui est un anneau lumineux dont le diamètre apparent D',

vu de l'œil à équiper, est notamment fonction du diamètre intérieur D du corps du dispositif. La largeur 1 de cet anneau 9 est égale au diamètre d de l'orifice circulaire de visée 4.

Lorsque l'axe visuel de l'œil et l'axe du dispositif sont confondus, selon x–x', c'est-à-dire lorsque le plan englobant la circonférence formé par le bord de la lentille de contact est perpendiculaire à l'axe x–x' du dispositif, la vision qu'a l'œil au moment du contact entre l'œil et la lentille est la suivante:

Au centre, l'orifice circulaire 4 de visée se détache sur l'opercule 3 légèrement translucide du fond du corps cylindrique et cet opercule 3 est lui-même couronné par l'anneau lumineux 9.

L'orifice de visée 4, l'opercule 3 du fond du corps cylindrique 1 et l'anneau lumineux 9 sont situés dans un même perpendiculaire à l'axe du dispositif.

Si, lors de l'approche de l'ensemble vers l'œil, l'axe de l'œil et l'axe du dispositif forment un angle entre eux, l'anneau lumineux 9 prend une forme plus ou moins elliptique fonction de l'importance de cet angle. L'orientation du dispositif doit être modifiée jusqu'à ce que l'image de l'anneau lumineux 9 obtenue soit circulaire. On peut alors procéder au contact entre la lentille et l'œil. De cette manière, on a bien un contrôle mathématique, découlant des lois de l'optique, du parallélisme indispensable entre la cornée et la lentille, d'où un succès assuré de la pose de la lentille.

## Revendications

1. Dispositif pour la pose d'une lentille de contact sur l'œil comprenant un corps cylindrique (1) terminé, à une extrémité, par un support (6) adapté à recevoir la lentille (8) à poser et, à son extrémité opposée, par un opercule (3) au centre duquel est percé un orifice (4) laissant passer la lumière ambiante, caractérisé en ce que la paroi interne du corps cylindrique (1) est réfléchissante.

2. Dispositif selon la revendication 1, caractérisé en ce que la paroi interne est revêtu d'un dépôt réfléchissant (2).

3. Dispositif selon la revendication 1, caractérisé en ce que la paroi interne est doublée d'un matériau réfléchissant (2).

4. Dispositif selon la revendication 1, caractérisé en ce que la matière formant le corps cylindrique (1) est naturellement réfléchissante.

## Patentansprüche

1. Einrichtung zum Einsetzen einer Kontaktlinse in das Auge mit einem zylindrischen Körper (1), der an einem Ende mit einer Stütze (6), die die einzusetzende Linse (8) aufnehmen kann, und an seinem gegenüberliegenden Ende mit einer Abdeckung (3) endet, in deren Mitte eine Öffnung (4) gebohrt ist, durch die Umgebungslicht einfallen kann, dadurch gekennzeichnet, dass die Innenwandung des zylindrischen Körpers (1) reflektierend ist.

2. Einrichtung nach Anspruch 1, dadurch gekennzeichnet, dass die Innenwandung mit einem reflektierenden Niederschlag (2) überzogen ist.

3. Einrichtung nach Anspruch 1, dadurch gekennzeichnet, dass die Innenwandung mit einem reflektierenden Material (2) ausgekleidet ist.

4. Einrichtung nach Anspruch 1, dadurch gekennzeichnet, dass das den zylindrischen Körper (1) bildende Material natürlich reflektierend ist.

**Claims**

1. Device for placing a contact lens in the eye comprising a cylindrical body (1) ending, at one end, in a support (6) adapted for receiving the lens (8) to be placed and, at its opposite end, in a cap (3) in the center of which is pierced an orifice (4) letting through the ambient light, characterized in that the internal wall of the cylindrical body (1) is reflecting.

2. Device according to claim 1, characterized in that the internal wall is coated with a reflecting deposit (2).

3. Device according to claim 1, characterized in that the internal wall is lined with a reflecting material (2).

4. Device according to claim 1, characterized in that the material forming the cylindrical body (1) is naturally reflecting.